# EUROPEAN PATENT APPLICATION

(11) **EP 3 175 839 A1**
(43) Date of publication of application: **07.06.2017**
(21) Application number: 15197494.6
(22) Date of filing: 02.12.2015
(51) Int. Cl.: A61K 8/73, A61Q 19/00, A61K 8/97

(54) **SKIN CARE COMPOSITION**

(71) Applicant: Wild & Natural Ibiza Cosmetics Corp. S.L., 07840 Santa Eulalia (ES)
(72) Inventor: LANWEHR, Christoph, 07840 Santa Eulalia (ES); GARCIA, Alain, 07800 Ibiza (ES)
(74) Representative: Hartz, Nikolai

(57) **Abstract**

Aqueous skin-care composition for external use, comprising an emulsion of
(a) papaya crude oil;
(b) *Cardiospermum halicacabum* extract; and
(c) an emulsifying agent.

## Description

The present invention relates to an aqueous skin-care composition for external use, which is useful for therapeutic and cosmetic applications. Specifically, the aqueous skin-care composition for external use of the present invention shows excellent pharmaceutical effects including analgesic, anti-inflammatory, antioedemic and antipuritic effects. Moreover, the aqueous skin-care composition for external use of the present invention shows excellent cosmetic effects by moisturizing the skin, preventing skin roughness, and reducing skin dullness.

The aqueous skin-care composition may be used in particular for the treatment of acute skin conditions such as jellyfish burns, sun burn, and skin injuries including mosquito bites, and burn injuries, as well as chronic skin conditions such as psoriasis, acne, dermatitis, atopic skin, eczema, or even anti-ageing applications.

Finally, the aqueous skin-care composition of the present invention may be used as a cream base for the preparation of further pharmaceutical or cosmetic compositions.

### Background of the invention

Jellyfish stings are a common problem for people swimming, wading or diving in seawaters. Jellyfish stings are the result of the long tentacles trailing from the jellyfish body which inject a person with venom from thousands of microscopic barbed stingers. Most often jellyfish stings result in immediate pain and red, irritated marks on the skin. Common signs and symptoms of jellyfish stings include burning, prickling, stinging pain, a "print" of the tentacles' contact with the skin, in the form of brown or purplish tracks on the skin, itching, swelling, tingling and numbness, and throbbing pain that radiates up a leg or an arm. If left untreated, severe oedema and inflammation may result which prevent the healing of the skin for weeks. Conventional treatments are based on the cleansing and disinfection of the skin followed by the application of cortisone preparations and antibiotics.

Sunburn is a form of radiation burn that affects the skin. Typically, there is initial redness (erythema), followed by varying degrees of pain, proportional in severity to both the duration and intensity of exposure. Other symptoms can include oedema, itching, peeling skin, rash, nausea, fever, chills, and syncope. Sunburns may be first- or second-degree burns. Non-steroidal anti-inflammatory drugs (such as ibuprofen or naproxen), and aspirin may decrease redness and pain. Topical steroids (such as hydrocortisone cream) do not help with sunburns. Applying soothing lotions that contain aloe vera to the sunburn areas may have a limited effect. However, an acceleration of the healing process is not available.

Psoriasis is an autoimmune disease characterized by patches of abnormal skin. These skin patches are typically red, itchy, and scaly. They may vary in severity from small and localized to complete body coverage. Plaque psoriasis typically presents with red patches with white scales on top. Areas of the body most commonly affected are the back of the forearms, shins, around the belly button, and the scalp. Guttate psoriasis has drop shaped lesions. Pustular psoriasis presents with small non-infectious pus filled blisters. Inverse psoriasis forms red patches in skin folds. Erythrodermic psoriasis occurs when the rash becomes very widespread, and can develop from any of the other types. Psoriasis appears to be a genetic disease which is triggered by environmental factors. There is no cure for psoriasis. However, various treatments can help control the symptoms such as steroid creams, vitamin D3 cream, ultraviolet light, and immune system suppressing medications such as methotrexate.

Dermatitis is inflammation of the skin which is characterized by dryness and recurring skin rashes including itchy, erythematous, vesicular, weeping, and crusting patches. Treatment is typically with moisturizers and steroid creams.

### Summary of the invention

The objective of the present invention is to provide a skin-care composition for external use which is useful for therapeutic and cosmetic applications and which may be used to treat or prevent acute skin disorders such as jellyfish stings or sunburn as well as chronic skin disorders such as psoriasis, and which has good cosmetic effects.

The object of the present invention is attained according to the claims. The present invention provides an aqueous skin-care composition for external use, comprising an emulsion of
(a) papaya crude oil;
(b) *Cardiospermum halicacabum* extract; and
(c) an emulsifying agent.

Moreover, the present invention provides an aqueous skin-care composition comprising an emulsion of
(a) papaya crude oil;
(b) *Cardiospermum halicacabum* extract; and
(c) an emulsifying agent,
for use in the treatment or prevention of jellyfish sting, sun burn, psoriasis, dermatitis, and acne.

The present inventors have found that hydrophilic extracts of *Cardiospermum halicacabum* in combination with hydrophobic papaya crude oil may give rise to unexpected benefits when externally used in the cosmetic or therapeutic treatment of skin. The combination may show synergistic effects on regulating the skin condition, and in particular on improving skin tone, skin texture and skin barrier function. The synergistic effects may be obtained by the potentialisation of the effects of the hydrophilic extract of *Cardiospermum halicacabum* by the hydrophobic papaya crude oil component in an aqueous emulsion. Moreover, the combination of the hydrophilic extract of *Cardiospermum halicacabum* and the hydrophobic papaya crude oil component in an aqueous emulsion provides further effects which cannot be obtained by either the hydrophilic extract of *Cardiospermum halicacabum* or the hydrophobic papaya crude oil component alone.

Furthermore, the present invention is based on the recognition that an emulsion of papaya crude oil, *Cardiospermum halicacabum* extract, and an emulsifying agent has an excellent pharmaceutical effect including analgesic, anti-inflammatory, antioedemic and antipuritic effects. Moreover, the aqueous skin-care composition for external use of the present invention has an excellent cosmetic effect by moisturizing the skin, preventing or improving skin roughness, reducing skin dullness, improving pigmentation, treating acnes, improving wrinkles, improving oily skin, improving dry skin, diminishing the appearance of pores, treating scarring, treating a burn injury, sterilizing the skin, killing mites on the skin, and improving skin texture.

### Detailed description of the invention

The term "hydrophilic" in relation to a material means that that material is more than 10% soluble in water by weight at standard temperature and pressure. The term "hydrophobic" as used in relation to a material means that that material is less than 1% soluble in water by weight at standard temperature and pressure.

The present invention provides an aqueous skin-care composition for external use. The aqueous skin-care composition may be formulated as a lotion having a low viscosity, or as a cream having a higher viscosity.

The aqueous skin-care composition for external use comprises an emulsion which is a fluid system in which liquid droplets are dispersed in a liquid. The droplets as the dispersed phase comprise preferably papaya crude oil as a hydrophobic phase.

The liquid as the continuous phase comprises preferably hydrophilic *Cardiospermum halicacabum* extract and water. Accordingly, the aqueous skin-care composition for external use is preferably an oil-in-water emulsion.

Alternatively, the amount of water may be reduced so as the droplets comprise preferably *Cardiospermum halicacabum* extract and water, and the liquid comprises preferably papaya crude oil, and an emulsifying agent. Accordingly, the aqueous skin-care composition for external use may be a water-in-oil emulsion.

The oily phase of the emulsion comprises papaya crude oil (a). Preferably, *carica papaya* L. (papaya) seed oil is used. Papaya crude oil (a) is a hydrophobic substance having very low miscibility with water or with hydrophilic *Cardiospermum halicacabum* extract. Papaya crude oil (a) preferably contains papain, also known as papaya proteinase I, peptidyl peptide hydrolase, E.C.-No.3.4.22.2, CAS-No.9001-73-4 which is a cysteine protease enzyme. Papain EC 3.4.22.2 hydrolyzes a wide variety of proteins and peptides. It has also esterase, thiolesterase, transesterase and transamidase activity.

Papaya crude oil (a) may provide a synergistic effect when combined with *Cardiospermum halicacabum* extract in an emulsion further containing an emulsifying agent. Papaya crude oil (a) contains papain at low concentrations which is essential for avoiding allergic reactions of the skin. The aqueous skin-care composition preferably contains 1 to 40 percent by weight based on the total weight of the composition of papaya crude oil (a). More preferably, the aqueous skin-care composition preferably contains 5 to 30 percent by weight based on the total weight of the composition of papaya crude oil (a). It was found that the emulsion according to the present invention does not result in an allergic reaction when the specific papain-containing papaya crude oil (a) is used.

The oily phase may further comprise additional oily components useful or conventional in the cosmetic field for this purpose in an amount which does not impair the effect of the present invention. Specific examples of additional oily components are fats and oils including cacao butter, horse fat, beef tallow, mutton tallow, hardened beef tallow, lard, beef bone tallow, Japanese core wax, hardened oil, Japanese wax, and hardened castor oil. Furthermore, the oily phase may also comprise waxes including bees wax, candelilla wax, cotton wax, carnauba wax, bayberry wax, tree wax, whale wax, montan wax, bran wax, lanolin, kapok wax, lanolin acetate, liquid lanolin, sugar cane wax, lanolin fatty acid isopropyl ester, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, and POE hydrogenated lanolin alcohol ether. Furthermore, the oily phase may also comprise synthetic ester oils or natural ester oils. As synthetic ester oils, isopropyl myristate, cetyl octanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxy stearate, ethylene glycol di-2-ethyl hexanoate, di-penta erythritol fatty acid ester, N-alkyl glycol monoiso stearate, neopentyl glycol dicaprate, glycerol di-2-heptyl undecanoate, diisostearyl malate, trimethyrol propane tri-2-ethyl hexanoate, tetra-2-ethyl hexanoate pentaerythritol, trimethyrol propane triisostearate, glycerol tri-2-ethyl hexanoate, glycerol trioctanoate, glycerol triisopalmitate, trimethyrol propane triisostearate, cetyl 2-ethylhexanoate, 2-ethylhexyl palmitate, glycerol trimyristate, glyceride tri-2-heptyl undecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate and triethyl citrate may be mentioned. As natural ester oils, avocado oil, camellia oil, turtle oil, macadamia nut oil, corn oil, mink oil, olive oil, rapeseed oil, egg yolk oil, sesame oil, wheat germ oil, southern piece oil, castor oil, linseed oil, safflower oil, cotton seed oil, perilla oil, soybean oil, groundnut oil, torreya oil, rice bran oil, chinese wood oil, jojoba oil, germ oil, triglycerol, trioctane acid glycerol and triiso palmitic acid glycerol may be mentioned.

The emulsion further comprises *Cardiospermum halicacabum* extract (b). Preferably, *Cardiospermum halicacabum* extract is CAS No. 89958-27-0 or EC No. 289-651-1. Preferably, the extract is obtained by aqueous ethanolic extraction of flower, leaf and vine of *Cardiospermum halicacabum.* The *Cardiospermum halicacabum* extract (b) may contain 40 to 80 percent by weight, preferably 50 to 70 percent by weight, and more preferably about 60 percent by weight of ethanol.

The aqueous skin-care composition preferably contains 1 to 50 percent by weight based on the total weight of the composition of cardiospermum extract (b). More preferably, the aqueous skin-care composition preferably contains 5 to 40 percent by weight based on the total weight of the composition of cardiospermum extract (b).

Preferably, weight ratio of papaya crude oil and cardiospermum extract (a)/(b) in the composition is 0.1 to 10, more preferably 0.5 to 5, still more preferably 0.7 to 2.

The emulsion further comprises an emulsifying agent (c). The emulsifying agent may comprise one or more emulsifiers. The emulsifiers are not particularly limited. Glycerides and/or any anionic, cationic, zwitterionic or polymeric emulsifyer may be used. A suitable mixture of two or more emulsifier compounds may be used.

Suitable glyceride emulsifiers are hydrogenated palm glyceride and hydrogenated castor oil. The anionic emulsifier may be selected from stearic acid and palmitic acid. The cationic emulsifier may be selected from cetyl trimethylammonium chloride, cetylpyridinium chloride, benzethonium chloride, hydroxypropyl guar hydroxypropyl trimonium chloride and guar hydroxypropyl trimonium chloride. The zwitterionic emulsifier may be selected from a lecithin, cocamidopropyl betaine, dodecyl betaine and phosphatidylcholine. In use, the net charge of the zwitterionic emulsifier is controlled through pH to yield either a net negative (anionic) or net positive (cationic) charge. The polymeric emulsifier may be selected from alginate, carboxymethylamylose, carboxymethylcellulose, carboxymethyldextran, carrageenan, cellulose sulphate, chondroitin sulphate, chitosan sulphate, dextran sulphate, gum arabic, gellan gum, heparin, hyaluronic acid, pectin, amidated pectins, xanthan gum, proteins and glycoproteins. Preferably, the emulsifying agent is polysaccharide, preferably xanthan gum. The aqueous skin-care composition for external use may contain 0.05 to 10 percent, preferably from 0.1 to 8 percent by weight and more preferably from 1 to 7 percent by weight emulsifyer based on the total weight of the composition.

The aqueous skin-care composition for external use contains water. The water may be added as pure water or as a solution or dispersion of one or more components dissolved or dispersed therein. In particular, a herbal distillate or hydrolate may be used which are aqueous products of distillation. A hydrolate is a colloidal suspensions of essential oils as well as water-soluble components obtained by steam distillation or hydrodistillation from plants/herbs whereby the watery distillate and not the essential oil is used. Preferably, a hydrolate of *Rosmarinus officinalis* is used as an aqueous component. A hydrolate of *Rosmarinus officinalis* is a colloidal suspension of essential oils as well as water-soluble components obtained by steam distillation or hydrodistillation from leafs, flowers and/or stems of *Rosmarinus officinalis*. The aqueous skin-care composition for external use may contain 5 to 40 percent, preferably from 10 to 30 percent by weight and more preferably from 15 to 25 percent by weight water or hydrolate based on the total weight of the composition.

According to a preferred embodiment, the aqueous skin-care composition for external use according to the present invention further comprises moringa seed oil. Moringa seed oil is preferably *Moringa oleifera* seed oil obtained by cold pressure of the seeds of the tree *Moringa oleifera.* LIPOFRUCTYL® MO LS 9305 (GMZ Inc. West Chester, Ohio) is a commercial product which may be used for the purposes of the present invention. The aqueous skin-care composition for external use according to the present invention may comprise from 5 to 45 percent by weight, preferably from 10 to 40 percent by weight and more preferably from 15 to 35 percent by weight *Moringa oleifera* seed oil, based on the total weight of the composition.

According to a preferred embodiment, the aqueous skin-care composition for external use according to the present invention further comprises *Aloe ferox* plant material. Aloe ferox is a species of arborescent aloe. The *Aloe ferox* plant material is preferably *Aloe ferox* leafs, in particular the gel which is obtainable by peeling a leaf and blending the clear gel in a blender. The aqueous skin-care composition for external use according to the present invention may comprise from 5 to 45 percent by weight, preferably from 10 to 40 percent by weight and more preferably from 15 to 35 percent by weight *Aloe ferox* plant material, based on the total weight of the composition.

An antioxidant may be added to the oil phase component of the aqueous skin-care composition of the present invention. Suitable antioxidants include ascorbic acid, acetyl cysteine, ascorbic acid polypeptide, ascorbyl dipalmitate, ascorbyl methylsilanol pectinate, ascorbyl palmitate, ascorbyl stearate, BHA, BHT, t-butyl hydroquinone, cysteine, cysteine HCI, diamylhydroquinone, di-t-butylhydroquinone, dicetyl thiodipropionate, dioleyl tocopheryl methylsilanol, disodium ascorbyl sulfate, distearyl thiodipropionate, ditridecyl thiodipropionate, dodecyl gallate, erythorbic acid, esters of ascorbic acid, ethyl ferulate, ferulic acid, gallic acid esters, hydroquinone, isooctyl thioglycolate, kojic acid, magnesium ascorbate, magnesium ascorbyl phosphate, methylsilanol ascorbate, natural botanical anti-oxidants such as green tea or grape seed extracts, nordihydroguaiaretic acid, octyl gallate, phenylthioglycolic acid, potassium ascorbyl tocopheryl phosphate, potassium sulfite, propyl gallate, quinones, rosmarinic acid, sodium ascorbate, sodium bisulfite, sodium erythorbate, sodium metabisulfite, sodium sulfite, superoxide dismutase, sodium thioglycolate, sorbityl furfural, thiodiglycol, thiodiglycolamide, thiodiglycolic acid, thioglycolic acid, thiolactic acid, thiosalicylic acid, tocophereth-5, tocophereth-10, tocophereth-12, tocophereth-18, tocophereth-50, tocopherol, tocophersolan, tocopheryl acetate, tocopheryl linoleate, tocopheryl nicotinate, tocopheryl succinate, and tris(nonylphenyl)phosphite. Preferably, ascorbic acid and tocopherol, is added. The amount of addition is usually 0.001 to 5.0 percent by weight, more suitably 0.01 to 3.0 percent by weight based on the total weight of the composition.

The aqueous skin-care composition for external use according to the present invention may further comprise at least one humectant. Preferably, the humectant comprises polyhydric alcohols. More preferably, the humectant comprises glycerine, propylene glycol, dipropylene glycol, polypropylene glycol, polyethylene glycol, sorbitol, hydroxypropyl sorbitol, hexylene glycol, 1,3-butylene glycol, 1,2,6-hexanetriol, ethoxylated glycerin, propoxylated glycerin or mixtures thereof. The aqueous skin-care composition for external use according to the present invention may comprise from 0.1 to 30%, preferably from 5% to 25% and more preferably from 7% to 15% humectant by weight of the composition.

The aqueous skin-care composition for external use according to the present invention may further comprise at least one thickener. Thickeners which may be employed according to the invention comprise C₁₂₋₂₄ fatty acids; C₁₀₋₃₀ fatty alcohols such as cetyl alcohol; particulate thickeners, such as hydrated colloidal aluminium silicate and magnesium aluminium silicate; carboxyvinyl polymers; sodium acrylate copolymers and hydrophobically modified sodium acrylate copolymers; polyacrylamide copolymers; polyacrylates, such as polyacrylate-13; acrylamide/ammonium acrylate copolymer; ammonium acryloyldimethyl taurate and ammonium acryloyldimethyl taurate crosspolymers and copolymers and hydrophobically modified ammonium acryloyldimethyl taurate copolymers. The aqueous skin-care composition according to the invention may comprise from 0.01% to 15%, preferably from 0.1% to 10% and more preferably from 1% to 7% thickener by weight of the composition.

A variety of additional optional ingredients may be incorporated into the compositions of the present invention. Non-limiting examples of these additional ingredients include additional skin care actives such as bisabolol, dialkanoyl hydroxyproline compounds, farnesol, flavonoids, guanidine (e.g., amino guanidine), N-acyl amino acid compounds, peptides, phytantriol, phytosterols, salicylic compounds, urea as well as compounds such as anti-acne compounds (e.g. resorcinol, erythromycin); skin soothing and healing agents; anti-wrinkle/anti-atrophy actives; conditioning agents; anti-inflammatory agents; skin lightening agents; antimicrobial/antibacterial/antifungal actives; chelators and sequestrants; and agents suitable for aesthetic purposes such as essential oils, fragrances, skin sensates, opacifiers, aromatic compounds (e.g., clove oil, menthol, camphor, eucalyptus oil, and eugenol), preservatives, or mixtures thereof. Preservatives may be selected from methyl paraben, propyl paraben, and p-chlorocresol.

In still another embodiment of the present invention the aqueous skin-care composition comprising an emulsion of
(a) papaya crude oil;
(b) *Cardiospermum halicacabum* extract; and
(c) an emulsifying agent
may be an aqueous cream base. Accordingly, cream formulations for topical use may be prepared by employing, for example, one or more active phamaceutical ingredients or herbal drugs which are from natural origin, and incorporating them into a cream base of the present invention. The cream base of the present invention optionally further comprises an ingredient selected from a group comprising a preservative, a buffering agent, an anti-oxidant, a chelating agent, and a humectant, or any combination thereof.

Accordingly, the present invention provides a new pharmaceutical preparation as a topical vehicle comprising an active agent dissolved in a biphasic lipophilic/hydrophilic cream base in the form of a biphasic emulsion cream. The system of the present invention has an advantage in being compatible with epidermal chemistry to enable ready transfer of any additional active ingredient from the vehicle to the skin while at the same time providing the beneficial effects of the combination of papaya crude oil and *Cardiospermum halicacabum* extract. The percutaneous transfer of an active substance from the base into the skin, takes place virtually completely so that a known amount of active ingredient as contained in the dose of preparation applied to the skin becomes bioavailable. The balanced hydrophilic/lipophilic vehicle does not form occlusive dressings on the skin and may easily be spread to form a uniform film to cause intimate contact at the skin interface. Through the use of the cream preparations of the present invention, both polar and non-polar active substances may be applied to the skin.

A preferred generic embodiment of the aqueous skin-care composition is as follows:

| Ingredient | Preferred Range Percent by weight | More Preferred Range Percent by weight |
|---|---|---|
| Papaya Seed Oil | 1-40 | 5-30 |
| *Cardiospermum halicacabum* extract | 1 -50 | 5-40 |
| Emulsifying agent | 0.05 - 10 | 1-7 |
| *Moringa oleifera* Seed Oil | 0-40 | 10-30 |
| *Aloe ferox* Leaf Juice | 0-40 | 10-30 |
| *Rosmarinus officinalis L*. Hydrolate | 0-60 | 10-50 |
| Salt (NaCl) | 0-10 | 0.5-5 |
| Thickening agent | 0-10 | 1-7 |
| Antioxidants | 0-5 | 0.1-3 |

The present invention further provides an aqueous skin-care composition comprising an emulsion of
(a) papaya crude oil;
(b) *Cardiospermum halicacabum* extract; and
(c) an emulsifying agent,
for use in the treatment or prevention of jellyfish stings, sun burn, and skin injuries including mosquito bites, and burn injuries.

The present invention further provides an aqueous skin-care composition comprising an emulsion of
(a) papaya crude oil;
(b) *Cardiospermum halicacabum* extract; and
(c) an emulsifying agent,
for use in the treatment or prevention of psoriasis, acne, dermatitis, atopic skin, eczema.

In still another embodiment, the aqueous skin-care composition of the present invention may be used as aqueous cream base.

The present invention further provides a process for preparing an aqueous skin-care composition, which comprises emulsifying a mixture of
(a) papaya crude oil;
(b) *Cardiospermum halicacabum* extract; and
(c) an emulsifying agent.

### Mode of administration

An aqueous skin-care composition for external use is provided for topical application to the skin. For a treatment of an acute skin condition the composition of the present invention is applied every two to four hours, whereby the injured portion of the skin is treated with a composition according to the invention for a duration of about two to five days by applying a thin layer to the portion of the skin in need thereof, and massaging the cream into the skin. For a treatment of a chronic condition, the composition of the present invention is applied once to five times daily by applying a thin layer and massaging the cream into the skin. With regard to a method of application, in the case of a liquid preparation, all the physically possible methods such as spraying, patching, dipping and masking can be used.

### Form of administration

The form of the external composition for the skin is not particularly limited, and it can be in any form for an external preparation including a an ointment, an emulsion, a cream, a gel, a facial mask, and a dispersion liquid.

According to a specific embodiment, the present invention provides a kit including the aqueous skin-care composition for external use of the present invention. Containers of the kits can include a bottle, a tube, a dispenser, a package, a compartment, or other types of containers, into which the aqueous skin-care composition may be placed. The containers can dispense a pre-determined amount of the aqueous skin-care composition. The composition can be dispensed in a spray, an aerosol, or in a liquid form or semi-solid form. The containers can have spray, pump, or squeeze mechanisms. The container can include indicia on its surface. The indicia, for example, can be a word, a phrase, an abbreviation, a picture, or a symbol. Where there is more than one component in the kit (they may be packaged together). The kit of the present invention may further comprise a tool or instrument for treating the skin and/or for applying the aqueous skin-care composition of the present invention. According to a preferred embodiment, the kit includes a tool for removing and cleaning remainder of the tentacles of a jellyfish from the skin. Preferably, the tool is a scraper having an edge which is suitable for scraping tentacles for avoiding secondary activation of nematocysts. The scraper may have the shape of a credit-card, for example. The kits of the present invention also can include a container housing the components in close confinement for commercial sale. Such containers may include injection or blow-molded plastic containers into which the desired bottles, dispensers, or packages are retained. A kit can also include instructions for employing the kit components as well the use of any other compositions, compounds, agents, ingredients, or objects not included in the kit. Instructions may include variations that can be implemented. For example, the instructions can include an explanation of how to apply, use, and maintain the products or compositions.

### Formulation Example 1 (Cream Base)

The following ingredients were thoroughly mixed in order to provide a cream base.
50 % *Carica Papaya* crude oil,
30 % *Rosmarinus officinalis* hydrolate,
10 % *Cardiospermum Halicacabum* Flower/Leaf extract,
5 % hydrogenated palm glyceride
5 % cetyl alcohol

### Example 2 (Cream)

A cream was prepared by mixing the ingredients as shown in Table 1

**Table 1**

| Ingredient | Preferred Range Percent by weight | Example 2 Percent by weight |
|---|---|---|
| Papaya Seed Oil | 5-15 | 10 |
| *Cardiospermum Halicacabum* Mother Tincture | 5-15 | 10 |
| Hydrogenated Palm Glyceride | 0.05 to 10 | 4 |
| Xanthan gum | 0.05-15 | 0.3 |
| *Moringa Oleifera* Seed Oil | 10-30 | 20 |
| *Aloe Ferox* Leaf Juice | 10-30 | 20 |
| *Rosmarinus Officinalis L.* Hydrolate | 10-50 | 29 |
| Salt (NaCl) | 0 to 10 | 1 |
| Cetyl alcohol | 0 to 10 | 5 |
| Tocopherol | 0 to 5 | 0.3 |
| Ascorbic acid | 0 to 2 | 0.4 |

### Example 3

A 59 year old male patient suffered from stings by a jellyfish (*Pelagia nocticula*). As a regular swimmer, the patient has frequently been stung by jellyfish in the past. The latest jellyfish stings have had important implications due to hypersensitivity to venom of *Pelagia nocticula.* A previous incident two years earlier involving the arms triggered a major oedema affecting the elbow and required, in addition to topical corticosteroid cream, the administration of oral corticosteroid tablets for 10 days.

The skin injuries from the jellyfish sting at the forehead were immediately very painful. Within few minutes after the sting the following clinical signs appeared:
- An erythema resembling a red plate of about 7 cm in width on the forehead;
- An oedema wherein the red plate being raised from 1 to 2 mm;
- Itchy blisters in certain areas of the red plate.

Within the following hours, the following clinical signs appears which are indicating the power of this envenomation. Local burning pain and itching remain for 24 hours. Slight headache with moderate general weakness also noted during 24 hours. The hypersensitivity of the patient might explain the sharp pain felt as well as the headache during the first 24 hours and the general weakness as a result of the sting.

The following treatment was started 3 days after the incident:
Application every 3 hours of the aqueous skin-care composition for external use according to Example 2 for 1 day. In view of the quick and good evolution of the case, the aqueous skin-care composition for external use was applied only twice daily on the second and third days of treatment.

After the first 24 hours, local burning sensation, itching and headache disappeared. On the second day, the oedema and the local erythema decreased by 90%. On the fourth day the skin appearance was almost normal, whereby only a very discrete redness remains which disappears completely on the sixth day of the treatment. The other clinical signs have disappeared completely.

In view of the location of the sting on the forehead and the size of the injury as well as given the hypersensitivity of the patient, detrimental aesthetic effects due to e.g. persisting elongated brown spots of a length of 7 cm long could be expected, which are frequently observed after *Pelagia Noctcicula* stings. However, the aqueous skin-care composition for external use of the present invention provides very fast and good evolution with no aesthetic consequences even in the absence of steroid tablets which were necessary during a previous sting.

### Example 4

A 21 year old male patient was poisoned by a sea anemone. Lesion were located at the left forearm. The patient is treated three days after being poisoned.

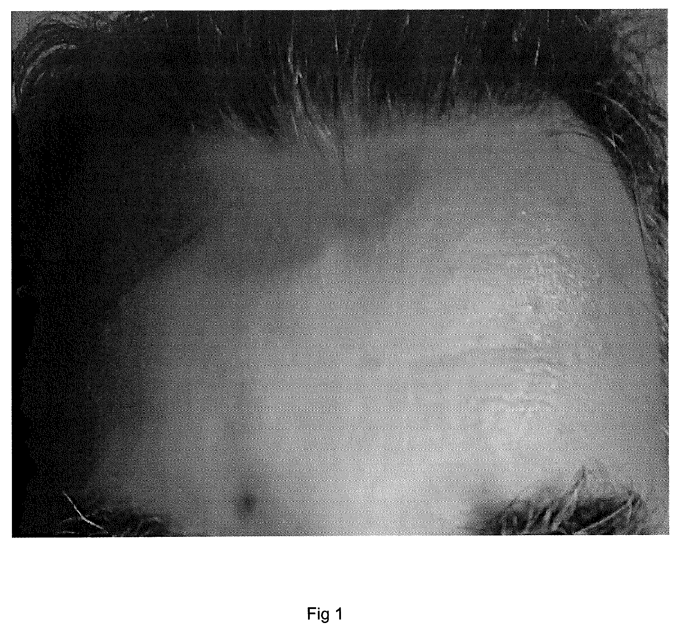

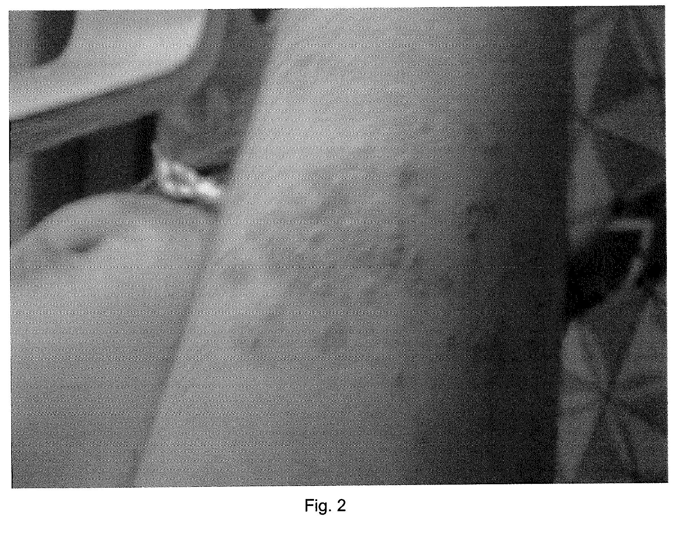

After three days without treatment, the patient had at the left forearm injuries of about 8 cm in diameter (Fig.2). An elevated plate presents an oedema and erythema as well as pustules at the contact sites. Scratch marks are also present and some pustules torn by scratching are oozing. The patient suffers from intense itching over the entire lesion.

The following treatment was started 3 days after the incident:
Application every 3 hours of the aqueous skin-care composition for external use according to Example 2 for 1 day. Facing the fast and good evolution of the case, the second and third days, the aqueous skin-care composition for external use was applied twice daily.

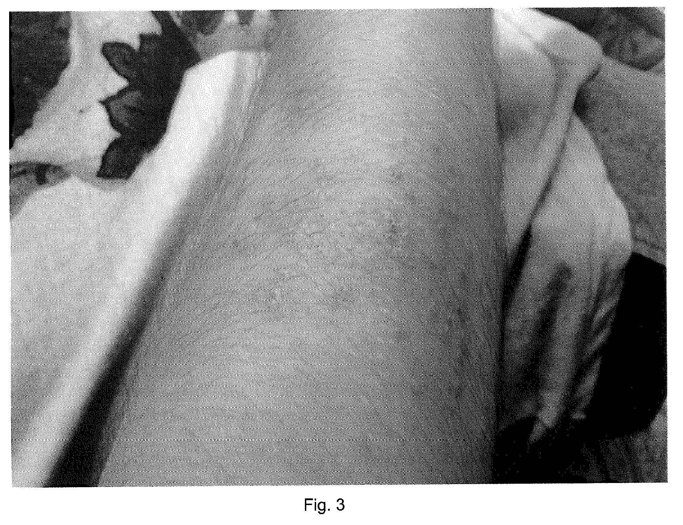

After the first 24 hours of treatment, the oedema had completely disappeared (Fig. 3). Erythematous lesions are dried, and continue in the process of healing. Papules and macules have disappeared by drying out. Moreover, the intense itching disappeared quickly. On the third day, the skin appearance is almost normal whereby only a few dark dots remain indicating an accelerated healing of the initial lesions.

## Claims

1. Aqueous skin-care composition for external use, comprising an emulsion of
(a) papaya crude oil;
(b) *Cardiospermum halicacabum* extract; and
(c) an emulsifying agent.

2. The aqueous skin-care composition for external use according to claim 1, wherein the weight ratio of papaya crude oil and cardiospermum extract (a)/(b) in the composition is 0.1 to 10.

3. The aqueous skin-care composition for external use according to claim 1 or 2, wherein the composition further comprises *Moringa oleifera* seed oil.

4. The aqueous skin-care composition for external use according to any one of the preceding claims, wherein the composition further comprises *Aloe ferox* plant material.

5. The aqueous skin-care composition for external use according to any one of the preceding claims, wherein the composition further comprises a hydrolate of *Rosmarinus officinalis.*

6. The aqueous skin-care composition for external use according to any one of the preceding claims, wherein the composition contains papaya crude oil (a) in an amount of 1 to 20 percent by weight based on the total weight of the composition.

7. The aqueous skin-care composition for external use according to any one of the preceding claims, wherein the composition contains cardiospermum extract (b) in an amount of 1 to 50 percent by weight based on the total weight of the composition.

8. The aqueous skin-care composition for external use according to any one of the preceding claims, wherein the composition contains an emulsifying agent (c) in an amount of 0.05 to 10 percent by weight based on the total weight of the composition.

9. The aqueous skin-care composition for external use according to any one of the preceding claims, wherein the emulsifying agent comprises a glyceride and/or xanthan gum.

10. The aqueous skin-care composition for external use according to any one of the preceding claims, which is a cream base.

11. Aqueous skin-care composition comprising an emulsion of
(a) papaya crude oil;
(b) *Cardiospermum halicacabum* extract; and
(c) an emulsifying agent,
for use in the treatment or prevention of jellyfish stings, sun burn, and skin injuries including mosquito bites, and burn injuries.

12. Aqueous skin-care composition comprising an emulsion of
(a) papaya crude oil;
(b) *Cardiospermum halicacabum* extract; and
(c) an emulsifying agent,
for use in the treatment or prevention of psoriasis, acne, dermatitis, atopic skin, eczema.

13. A process for preparing an aqueous skin-care composition, which comprises emulsifying a mixture of
(a) papaya crude oil;
(b) *Cardiospermum halicacabum* extract; and
(c) an emulsifying agent.

14. Kit for the treatment of jellyfish stings, which comprises
(i) an aqueous skin-care composition for external use according to any one of claims 1 to 9, and
(ii) a scraper.
